# EUROPEAN PATENT APPLICATION

(11) **EP 2 324 864 A1**
(43) Date of publication of application: **25.05.2011**
(21) Application number: 09811312.9
(22) Date of filing: 08.09.2009
(51) Int. Cl.: A61L 31/00, A61L 33/00, C08K 5/09, C08L 5/00, C08L 89/00, C08L 101/00

(54) **COMPOSITE MATERIAL COMPRISING HIGH-MOLECULAR-WEIGHT MATRIX AND LOW-MOLECULAR-WEIGHT ORGANIC COMPOUND AND PROCESS FOR PRODUCING SAME**

(30) Priority: 08.09.2008 JP 2008229442
(71) Applicant: National Institute for Materials Science, Tsukuba-shi, Ibaraki 305-0047 (JP)
(72) Inventor: TAGUCHI, Tetsushi, Tsukuba-shi Ibaraki 305-0047 (JP); TAKAYANAGI, Mariko, Tsukuba-shi Ibaraki 305-0047 (JP)
(74) Representative: Jansen, Cornelis Marinus
(86) International application number: PCT/JP2009/004432
(87) International publication number: WO 2010/026782

(57) **Abstract**

Previously reported composite materials comprising a hydrophilic high-molecular-weight matrix and a low-molecular-weight organic compound involve a problem in that: it is difficult to compound a high load of the low-molecular-weight organic compound in the high-molecular-weight matrix; and the thus produced composite material yields a high-molecular-weight matrix component that can form a thrombus when contacting with blood. It is now possible to produce a composite material having an antithrombogenic activity and a high content of a low-molecular-weight organic compound by the present invention comprising the steps of: crosslinking a polymer with an organic acid derivative which is used as a crosslinker in an organic solvent while, at the same time, embedding the low-molecular-weight organic compound therein; and substituting the organic solvent with water after the production of the high-molecular-weight matrix to effect precipitation and compounding of the low-molecular-weight organic compound in the high-molecular-weight matrix.

## Description

### TECHNICAL FIELD

The present invention relates to a composite material comprising a high-molecular-weight matrix and a low-molecular-weight organic compound, and a process for producing the same.
Priority is claimed from Japanese Patent Application No. 2008-229442, filed September 8, 2008, the content of which is incorporated herein by reference.

### BACKGROUND ART

Heretofore, composite materials comprising a synthetic polymer, a natural polymer, a cell growth factor, a drug, a calcium phosphate, and the like have been reported and applied to coatings of scaffolding materials for regenerative medication, and various kinds of solid substrates. For example, in the treatment of ischemic heart disease, which can be a cause of myocardial infarction, there has been used a drug-eluting stent (DES) in which a composite material comprising a high-molecular-weight matrix and a drug, being a low-molecular-weight organic compound, is coated over the surface of the stent. This DES makes it possible for the low-molecular-weight organic compound, such as a drug, embedded inside the high-molecular-weight matrix to be eluted *in vivo* and thereby suppress restenosis (for example, Patent Documents 1 and 2).

However, it is reported that, after the low-molecular-weight organic compound has been eluted, the remaining high-molecular-weight matrix brings about inflammatory reactions and thrombus formation, and furthermore inhibits endothelialization of the stent surface (for example, refer to Non-Patent Documents 1 and 2).

Patent Document 1 discloses that a water-absorptive polymer which contains a drug, being a low-molecular-weight organic compound, and which is capable of absorbing water weighing at least 100% or more of its own weight, is effective as DES. In particular, it is disclosed that the water-absorptive polymer material is preferably: a polyurethane-based elastomer which has a polyether chain, such as a polypropylene oxide chain and a polytetramethylene oxide chain, in the structure; or a polymer matrix or a polymer matrix-like material based on such an elastomer; so as to keep the stability of the physical strength *in vivo* (in paragraph [0012] lines 8 to 10). Moreover, the method employed for embedding the drug in the polymer matrix is an embedding method in which the absorbency of the polymer matrix is utilized by using a water soluble drug (in paragraph [0013] lines 1 to 3). The polymer matrix for use *in vivo* is preferably capable of releasing the drug, as well as being capable of eliminating from *in vivo* after the action.

Patent Document 2 proposes a base material which is coated by a polymer of as small a weight as possible. It is disclosed that: such a polymer matrix is preferably a lactic acid-glycolic acid copolymer; and that a drug, being a low-molecular-weight organic compound, and a polymer are dissolved in chloroform and thereafter dried in a vacuum at room temperature for 1 hour (in paragraph [0052] lines 1 to 5 and paragraph [0053] line 9).

Patent Document 3 discloses that water or an alcohol solution containing a drug being a low-molecular-weight organic compound, is homogeneously mixed in a PBS solution containing a biodegradable matrix-forming material and an enzyme for crosslinking the biodegradable matrix-forming material, to disperse the drug all over the solution (in paragraph [0028] lines 1 to 6).

Patent Document 1: Japanese Unexamined Patent Application, First Publication No. 2004-89233
Patent Document 2: Japanese Unexamined Patent Application, First Publication No. 2007-312987
Patent Document 3: Japanese Unexamined Patent Application, First Publication No. 2007-277217

Non-Patent Document 1: Xianyan Wang, Xiaohui Zhang, John Castellot, Ira Herman, Mark Iafrati, and David L. Kaplan: Biomaterials 2008; 29: 894-903.
Non-Patent Document 2: Han Hee Cho, Dong-Wook Han, Kazuaki Matsumura, Sadami Tsutsumi, and Suong-Hyu Hyon: Biomaterials 2008; 29: 884-893.

With previously reported composite materials comprising a hydrophilic high-molecular-weight matrix and a low-molecular-weight organic compound, the problem is that: it is difficult to compound a high load of the low-molecular-weight organic compound in the high-molecular-weight matrix; and when the produced composite material contacts with blood, the high-molecular-weight matrix component in the composite material can form a thrombus.

### DISCLOSURE OF INVENTION

The present invention provides a composite material comprising a high-molecular-weight matrix and a low-molecular-weight organic compound, wherein the composite material has the low-molecular-weight organic compound dispersed in the high-molecular-weight matrix, and the high-molecular-weight matrix is a polymer crosslinked with an organic acid derivative.

Moreover, the present invention provides a process for producing a composite material comprising a high-molecular-weight matrix and a low-molecular-weight organic compound, wherein the process comprises the steps of: crosslinking a polymer with an organic acid derivative in an organic solvent while, at the same time, embedding the low-molecular-weight organic compound therein; and substituting the organic solvent with water after the production of the high-molecular-weight matrix.

In the composite material of the present invention, it is preferable that the organic acid derivative has two or three carboxyl groups of a dicarboxylic acid or a tricarboxylic acid modified with electron attracting groups.

Preferably, the low-molecular-weight organic compound contained in the composite material of the present invention is a combination of a drug which is soluble in an aprotic polar organic solvent and a single type or a plurality of types of amino acids, and is poorly soluble in water.
Moreover, the composite material of the present invention preferably has the property of gradually releasing the low-molecular-weight compound.
Furthermore, the composite material of the present invention preferably has an antithrombogenic activity.

According to the present invention, it is possible to prepare a composite material having a high concentration of a low-molecular-weight organic compound by mixing the low-molecular-weight organic compound in a polymer solution and crosslinking the polymer with an organic acid derivative in which an active ester group is introduced. Moreover, it is also possible to provide a composite material having excellent biocompatibility and excellent antithrombogenic activity by using the polymer of the present invention. Furthermore, because the rate of releasing the low-molecular-weight organic compound from the high-molecular-weight matrix can be readily controlled by adjusting the crosslink density of the polymer of the present invention, the application as a composite material having excellent biocompatibility is possible.

### BRIEF DESCRIPTION OF THE DRAWINGS

FIG. 1 shows a change of the gel diameter in water at 4°C (0 min, 10 min, 1 h, 3 h, 6 h, and 24 h from the left).
FIG. 2 shows the amounts of Am80 released from matrices in the Example.
FIG. 3 shows the influence of Am80 embedded with an organic acid derivative concentration (5 mM), on the thrombus formation.
FIG. 4 shows the influence of Am80 embedded with an organic acid derivative concentration (20 mM), on the thrombus formation.
FIG. 5 shows the influence of Am80 embedded with an organic acid derivative concentration (100 mM), on the thrombus formation.
FIG. 6 shows a high-molecular-weight matrix prepared by including 100 mM AcTrpNH₂.
FIG. 7 shows a high-molecular-weight matrix prepared by including 100 mM AcTyrNH₂.
FIG. 8 shows a high-molecular-weight matrix prepared by including 100 mM FmocIleOH.
FIG. 9 shows a high-molecular-weight matrix prepared by including 100 mM FmocPheOH.
FIG. 10 shows a high-molecular-weight matrix prepared by including 100 mM FmocTrpOH.
FIG. 11 shows a high-molecular-weight matrix prepared by including 100 mM FmocSer(tBu)OH.
FIG. 12 shows a high-molecular-weight matrix prepared without including any amino acid derivative.

### BEST MODE FOR CARRYING OUT THE INVENTION

Hereunder is a description of an embodiment of the present invention. However, the present invention is in no way limited to this embodiment.

The term "composite material" used in the present invention refers to a material produced by mixing a polymer, an organic acid derivative, and a low-molecular-weight organic compound such as a drug. The material is compounded by a crosslinking reaction at a molecular level between the polymer and the organic acid derivative.

The polymer for use in the present invention can be exemplified by a single item, or a combination of a plurality of items, selected from: a protein, a glycosaminoglycan, a chitosan, a polyamino acid, and a polyalcohol. Preferred examples of the protein can include collagen, atelocollagen, alkali-treated collagen, gelatin, acid-treated gelatin, alkali-treated gelatin, keratin, serum albumin, egg albumin, recombinant albumin, hemoglobin, casein, globulin, fibrinogen, and derivatives thereof. More preferred are a collagen, a gelatin, and derivatives thereof. Desirably, the polymer for use in the present invention has an amino group in the molecule to be suitable for the crosslinking reaction with the organic acid derivative. Preferred examples of the glycosaminoglycan can include chondroitin sulfate, dermatan sulfate, hyaluronic acid, heparan sulfate, heparin, keratan sulfate, and derivatives thereof.

The concentration of the polymer in the mixture solution is not specifically limited, and is normally from 3 to 30 wt%, preferably from 7.5 to 15 wt%, more preferably from 10 to 15 wt%, and most preferably 15 wt%. If the concentration of the polymer is less than 7.5 wt%, the concentration of the organic acid derivative would be 20 mM or higher; in which case, the high-molecular-weight matrix cannot be formed. In other words, the crosslinked structure having a high crosslink density would not be maintained.

The organic acid derivative for use in the production of the composite material of the present invention is preferably a single type of, or a combination of a plurality of types of, modified products in which at least two carboxyl groups of any one or more items, selected from: citric acid, malic acid, oxaloacetic acid, tartaric acid cis-aconitic acid, 2-ketoglutaric acid, and derivatives thereof, are modified with N-hydroxysuccinimide, N-hydroxysulfosuccinimide, or a derivative thereof.

The suitable concentration of the organic acid derivative for the production of the composite material of the present invention is normally from 1 to 100 mM, preferably from 5 to 100 mM, more preferably from 5 to 80 mM, and most preferably 20 mM, when the concentration of the polymer is 15 wt%. If the concentration of the organic acid derivative is lower than 5 mM, the number of crosslink points between the polymer and the organic acid derivative would be too small to maintain the high-molecular-weight matrix structure. In addition, if the concentration of the organic acid derivative is higher than 100 mM, a structure in which the polymer and the organic acid are bound to each other would take place; in which case, the number of crosslink points decreases so much that the high-molecular-weight matrix structure can not be maintained. Moreover, the reaction time is preferably within 24 hours at 25°C. For example, if the organic acid derivative is added at 20 mM relative to the whole reaction solution, the time for forming the high-molecular-weight matrix would be 10 to 20 minutes at room temperature.

The low-molecular-weight compound of the present invention is preferably a single item, or a combination of a plurality of items, selected from: a drug which is poorly soluble in water, an amino acid, and a derivative thereof.

For the preparation of the composite material of the present invention, an aprotic polar solvent is used as the reaction solvent. As an example, N,N-dimethylsulfoxide (DMSO) can be used. For the removal of the reaction product (N-hydroxysuccinimide) and unreacted matters generated during the production of the high-molecular-weight matrix using the polymer and the organic acid derivative, it is possible to immerse the high-molecular-weight matrix in pure water to cause the removal by substitution. Moreover, when substituting the organic solvent held by the high-molecular-weight matrix into pure water, it is preferable to carry out the substitution at 0 to 10°C or lower temperature so as to suppress the dissolution of the embedded low-molecular-weight organic compound and to suppress the hydrolysis of the polymer.

The specific means for mixing these substances in the preparation of the mixture solution is not limited, although it is preferable to sufficiently and homogenously mix them by using an agitator such as a small-sized mixer.

Hereunder is a more detailed description of the present invention for each high-molecular-weight matrix with reference to the Examples. However, the present invention is not to be limited by these Examples.

### Example 1

### 1-1 Preparation of high-molecular-weight matrices without any embedded low-molecular-weight organic compound

The common production method for respective matrices shown in Table 1 is as follows.
The high-molecular-weight matrices were prepared at room temperature by varying the concentration of the organic acid derivative (TriSuccinimidyl Citrate: TSC) as shown in Table 1. The polymer (600 mg) was added with 10% lactic acid-containing DMSO (4.00 mL), by which a 15 w/v% solution was prepared. Next, the organic acid derivative was added to a 10% lactic acid-containing DMSO solution and mixed to give the final concentration of 20 mM, by which an organic acid derivative solution was prepared. The polymer solution and the organic acid derivative solution were mixed at a rate of 4:1, and quickly agitated with a pencil mixer for 30 sec. Then, the mixture was degassed by a centrifugal separator for 30 sec, yielding a polymer without an embedded drug. The high-molecular-weight matrices were formed within a predetermined time when the concentration of the organic acid derivative (TSC) was up to 100 mM.

**[Table 1]**

| Biodegradable polymer | | | | | | | |
|---|---|---|---|---|---|---|---|
| JNo | MNo | A solution | | B solution | | A:B | Drug content Concentration mM |
| | | Component | Concentration w/v % | Component | Concentration mM | | |
| 1 | 1 | Alc-DMSO | 15 | TSC DMSO | 5 | 4:1 | Not contained |
| | 2 | *"* | *"* | *"* | 10 | *"* | *"* |
| | 3 | *"* | *"* | *"* | 20 | *"* | *"* |
| | 4 | *"* | *"* | *"* | 40 | *"* | *"* |
| | 5 | *"* | *"* | *"* | 50 | *"* | *"* |
| | 6 | *"* | *"* | *"* | 70 | *"* | *"* |
| | 7 | *"* | *"* | *"* | 100 | *"* | *"* |
| 2 | 1 | Alc-DMSO | 15 | TSC-DMSO | 20 | 4:1 | 34.82 |
| | 2 | *"* | *"* | *"* | *"* | *"* | 348.2 |
| | 3 | *"* | *"* | *"* | *"* | *"* | 696.4 |
| | 4 | *"* | *"* | *"* | *"* | *"* | 1392.8 |
| 3 | 1 | Alc-DMSO | 15 | TSC-DMSO | 5 | 4:1 | 34.8 |
| | 2 | *"* | *"* | *"* | 20 | *"* | 34.8 |
| | 3 | *"* | *"* | *"* | 100 | *"* | 34.8 |
| 4 | 1 | Gelatin-DMSO | 15 | TSC-DMSO | 5 | 4:1 | Not contained |
| | 2 | *"* | *"* | *"* | 10 | *"* | *"* |
| | 3 | *"* | *"* | *"* | 20 | *"* | *"* |
| | 4 | *"* | *"* | *"* | 40 | *"* | *"* |
| | 5 | *"* | *"* | *"* | 50 | *"* | *"* |
| | 6 | *"* | *"* | *"* | 70 | *"* | *"* |
| | 7 | *"* | *"* | *"* | 100 | *"* | *"* |
| 5 | 1 | Gelatin-DMSO | 15 | TSC-DMSO | 5 | 4:1 | 34.8 |
| | 2 | *"* | *"* | *"* | 20 | *"* | 34.8 |
| | 3 | *"* | *"* | *"* | 100 | *"* | 34.8 |
| JNo: Number of Example | | | | A:B : Blending ratio between A solution and B solution | | | |
| MNo: Number of matrix | | | | | | | |
| A solution : Polymer solution | | | | Alc Alkali-treated collagen | | | |
| B solution : Organic acid derivative solution | | | | TSC : Organic acid derivative | | | |
| DMSO : N,N-dimethylsulfoxide (aprotic polar solvent) | | | | | | | |

### 1-2 Stability of high-molecular-weight matrices without any embedded low-molecular-weight organic compound

The thus produced high-molecular-weight matrices were immersed in pure water (5 mL) at 4°C or 37°C to examine the time course change of the high-molecular-weight matrices. Table 2 shows the state of the high-molecular-weight matrices of Example 1 (matrices 1 to 7) of Table 1 after one month. The high-molecular-weight matrices respectively absorbed water and so swelled that their sizes and weights were changed with time. Thereafter, shrinkage of these high-molecular-weight matrices was found, and disappearance of the high-molecular-weight matrices due to a hydrolysis reaction was observed.

**[Table 2]**

| Concentration of crosslinker/mM | Presence/absence of crosslinked body (4°C) | Presence/absence of crosslinked body (37°C) |
|---|---|---|
| 5 | Present | Absent |
| 10 | Present | Present |
| 20 | Present | Present |
| 40 | Present | Present |
| 50 | Present | Present |
| 70 | Present | Absent |
| 100 | Present | Absent |

When immersed at 37°C, the hydrolysis reaction was so promoted that the disruption of the high-molecular-weight matrices was accelerated. The high-molecular-weight matrices disappeared in about two weeks when the concentration of the organic acid derivative (TSC) was 5 mM or 100 mM, and in one month when the concentration was 70 mM.

The above-mentioned results suggest that it is necessary to remove DMSO, the reaction product, and unreacted matters with pure water at 4°C or lower temperature in the production of a polymer with an embedded drug, so as to suppress the elution of the drug and to form the high-molecular-weight matrix in which the polymer is stable.

Moreover, the high-molecular-weight matrix of JNo. 1-MNo. 3 of Table 1 was naturally dried and then heated at 80°C for 10 minutes. This was used as a sample for the evaluation of the degree of degradation and stability with an enzyme. As for the solution for the degradation reaction with an enzyme, a 0.1 wt% collagenase/PBS solution was prepared by adding a collagenase (Nacalai Tesque) to a 5 mM CaCl₂/PBS solution. This enzymatic reaction solution was matured at 37°C in advance before use for the reaction. The degradation rate was examined by the weight difference between before and after the matrix had been immersed in the collagenase/PBS solution and agitated at 37°C, or by the amino acid residue assay using a TNBS method.

Table 3 shows the degradation rate of the high-molecular-weight matrix under the presence of the collagenase. It was demonstrated that the enzymatic degradation proceeded with time.

**[Table 3]**

| Degradation time/min | Degradation rate (%) |
|---|---|
| 5 | 2.43 |
| 15 | 29.61 |
| 30 | 70.93 |
| 45 | 91.21 |
| 60 | 100 |

### Example 2

### 2-1 Investigation on compounding of high-molecular-weight matrix and low-molecular-weight organic compound

The compounding of the high-molecular-weight matrix (Alc) and the low-molecular-weight organic compound produced by 1-1 mentioned above was investigated.

### 2-2 Preparation of high-molecular-weight matrix containing low-molecular-weight organic compound

The matrix of JNo. 2-MNo. 1 of Example 2 shown in Table 1 was prepared. The polymer (600 mg) was added to DMSO (4.00 mL), by which a 15 w/v% solution was prepared. This solution was added with a low-molecular-weight organic compound (Tamibarotene: Am80) to give the final concentrations of 34.82, 348.2, 696.4, and 1392.8 mM and agitated, by which polymer/low-molecular-weight organic compound/DMSO mixture solutions were prepared. Next, the organic acid derivative was added to a DMSO solution and mixed to give the final concentration of 20 mM, by which an organic acid derivative solution was prepared. The polymer solution and the organic acid derivative solution were mixed at a rate of 4:1, and quickly agitated with a pencil mixer for 30 sec. Then, the mixture was degassed by a centrifugal separator for 30 sec, yielding the high-molecular-weight matrices containing the low-molecular-weight organic compound.
The high-molecular-weight matrices containing the low-molecular-weight organic compound were taken out from the reaction vessel, and immersed in 100 mL of pure water at 4°C or 37°C.

The formation of the thus prepared high-molecular-weight matrices having different Am80 concentrations took twice as long as the formation of the matrix without any embedded Am80. However, it was demonstrated that it was possible in all systems to form the high-molecular-weight matrices containing the low-molecular-weight organic compound.

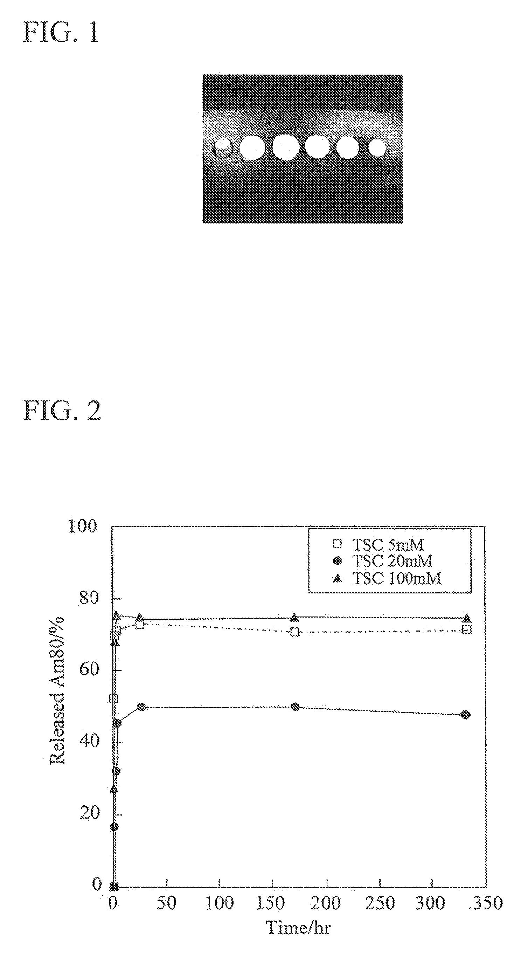

Next, the stability of the high-molecular-weight matrices was observed by weight and by visual check during the substitution of the solvent for the high-molecular-weight matrices mentioned above. As shown in FIG. 1 and Table 4, these high-molecular-weight matrices showed a tendency of absorbing water and swelling with time when immersed in pure water, and a tendency of shrinking, similar to the high-molecular-weight matrices without any embedded low-molecular-weight organic compound.

It was demonstrated that the structure was maintained in all high-molecular-weight matrices even if the low-molecular-weight organic compound was embedded therein.

**[Table 4]**

| Immersion time (min) | Gel diameter (mm) |
|---|---|
| 0 | 10 |
| 10 | 12.3 |
| 60 | 13.1 |
| 180 | 10.8 |
| 360 | 10.0 |
| 1440 | 7.7 |

### Example 3

### 3-1 Property of gradually releasing low-molecular-weight organic compound (Am80) from composite material

The high-molecular-weight matrices containing the low-molecular-weight organic compound prepared in 2-1 mentioned above were dried in a desiccator and then heated in an oven at 80°C for 10 min. These composite materials were used as samples for HPLC.

The thus produced high-molecular-weight matrices were each transferred in a 250 mL polyethylene container, and 200 mL of PBS (pH7.4) was added thereto. These were heated in an incubator at 37°C. The supernatant thereof was sampled in a time course manner, and 50 µL each was assayed by HPLC.

In the HPLC method, the Nacalai Tesque COSMOSIL Packed Column (Size: 4.6 I.D. × 150 mm; Type: SC₁₈-AR II ATERS) was used as the column, and a 5% HOAc/CH₃CN = 35/65 (v/v) aqueous solution was used as the eluent. The flow rate was 1.00 mL/min, and the column temperature was 40.0°C. The detection was conducted by UV-visible spectroscopy.

The elusion amount was calculated based on a calibration curve that had been formed in advance. Three kinds of drug/PBS (pH7.4) solutions were prepared within a concentration range from 1.14 µM to 569 µM, and assayed by HPLC. The condition for this assay was the same as the above.

When immersed in PBS (pH7.4 at 37°C), the thus produced composite materials absorbed the solvent and swelled to thereby take a slightly whitish color and then gradually became transparent from the periphery. The results of the Am80 release amount are shown in FIG. 2. Moreover, the numerical values of the release rates of FIG. 2 are shown in Table 5. Am80 was released little by little, and 49% was released in one day. From these results, it was shown that the composite materials produced by the above-mentioned technique were controllably releasing Am80 in a short time and were capable of releasing it over a long period.

**[Table 5]**

| Elapsed time/hr | Example 3 Matrix 1 | Example 3 Matrix 2 | Example 3 Matrix 3 |
|---|---|---|---|
| 0 | 0 | 0 | 0 |
| 1 | 53.2 | 17.3 | 27.9 |
| 3 | 69.0 | 32.8 | 67.3 |
| 5 | 70.9 | 44.9 | 75.7 |
| 24 | 73.2 | 32.8 | 75.4 |
| 168 | 70.7 | 49.0 | 75.9 |
| 336 | 71.3 | 46.9 | 75.4 |

### 3-2 Control of Am80 release amount by varying the crosslink density

The concentration of the organic acid derivative was varied at 5, 20, and 100 mM according to the proportions of Example 3 shown in Table 1 to prepare high-molecular-weight matrices in which 34.82 mM Am80 was embedded. The preparation method was the same as the above-mentioned technique. The produced disk-shaped high-molecular-weight matrices were immersed in pure water. Water was replaced every two hours, five times a day, and the immersion was carried out for three days. Thereafter, the high-molecular-weight matrices were taken out, dried in a desiccator, and then heated in an oven at 80°C for 10 min. These composite materials were used as samples for HPLC.

In the HPLC method, the Am80 elusion amount was calculated under the same conditions as for the above-mentioned method.

Table 6 shows the rates of releasing Am80 from the composite materials having different organic acid derivative concentrations. As shown above, the high-molecular-weight matrix containing 20 mM organic acid derivative showed a release ratio of 49%. On the other hand, it was demonstrated that the high-molecular-weight matrices containing 5 mM and 100 mM organic acid derivative respectively had release ratios of 70% and 75%.

**[Table 6]**

| Drug release rate of JNo 3 | | | | |
|---|---|---|---|---|
| MNo | | 1 | 2 | 3 |
| Elapsed time (hr) | 0 | 0 | 0 | 0 |
| | 1 | 53.2 | 17.3 | 27.9 |
| | 3 | 69.0 | 32.8 | 67.3 |
| | 5 | 70.9 | 44.9 | 75.7 |
| | 24 | 73.2 | 32.8 | 75.4 |
| | 168 | 70.7 | 49.0 | 75.9 |
| | 336 | 71.3 | 46.9 | 75.4 |

### Example 4

High-molecular-weight matrices using gelatin were prepared. As shown in Table 1, a 10% lactic acid-containing DMSO solution in which gelatin was dissolved, and a 10% lactic acid-containing DMSO solution (from 5 to 100 mM) in which the organic acid derivative was dissolved, were mixed at a ratio of 4:1.

When the concentration of the organic acid derivative (TSC) was 100 mM or lower, high-molecular-weight matrices using gelatin (using gelatin) were formed. When these high-molecular-weight matrices were immersed in pure water, the degree of swelling increased with time as shown in Table 7. The degree of swelling was the highest when the organic acid derivative concentration was 40 mM, and the degree of swelling was the lowest when the concentration was 20 mM.

**[Table 7]**

| Time (h) | TSC 10 mM | TSC 20 mM | TSC 40 mM |
|---|---|---|---|
| 0.5 | 9.497207 | 8.133896 | 12.21505 |
| 1 | 11.01397 | 9.016883 | 13.31586 |
| 2 | 12.49302 | 9.274026 | 13.80242 |
| 3 | 12.62709 | 9.375325 | 14.05511 |
| 5 | 12.92458 | 9.498701 | 14.17473 |
| 7 | 12.8338 | 9.407792 | 14.17473 |
| 10 | 12.76397 | 9.492208 | 14.24462 |
| 24 | 12.77235 | 9.427273 | 14.41935 |

### Example 5

Composite materials, in which a drug was embedded in the above-mentioned high-molecular-weight matrices using gelatin, were prepared. Am80 (61.2 mg, 174 µmol) was added to 4.00 mL of a 15 wt% Gelatin/DMSO solution and quickly dissolved. Thereafter, the mixture was degassed by a centrifugal separator, and then was added with a TSC/10% lactic acid-containing DMSO solution. This was agitated with a pencil stirrer mixer for 1 minute, then degassed by a centrifugal separator, and left still for one day. Thereby, a plate gel was produced. The produced plate gel was cut out in a circular shape having a diameter of 10 mm, and immersed in pure water (4°C, 50 mL) for three days. Thereafter, the plate was naturally dried and heated at 80°C. The composite materials were produced by varying the TSC concentration at 5 mM, 20 mM, and 100 mM. In the Am80 release test, 50 mL of 0.1M PBS (pH7.4) was previously measured and placed in a centrifugal tube. This was kept at 37°C in an incubator. The composite materials were immersed therein, and were sampled at certain times. The Am80 content thereof was assayed by HPLC.

In all cases, irrespective of the concentration of the organic acid derivative, composite materials were able to be produced. Similar results were given even when gelatin was used instead of the alkali-treated collagen.

**[Table 8]**

| Time (h) | TSC 5 mM | TSC 20 mM | TSC 100 mM |
|---|---|---|---|
| 5 | 66.957 | 38.087 | 71.93 |
| 7 | 66.865 | 44.57 | 77.121 |
| 12 | 74.403 | 63.615 | 80.298 |
| 24 | 72.433 | 75.005 | 78.078 |
| 168 | 73.172 | 69.939 | 79.529 |
| 336 | 73.595 | 69.667 | 78.874 |
| 504 | 70.665 | 104.01 | 78.906 |
| 672 | 69.145 | 62.527 | 74.416 |

### Example 6

### Evaluation of antithrombogenic activity of composite materials

The composite materials of Example 3 shown in Table 1 were evaluated for the antithrombogenic activity. According to the production method of the disk-shaped high-molecular-weight matrix mentioned above, matrices having organic acid derivative (TSC) concentrations of 5, 20, and 100 mM were produced. 1 mL of rat arterial blood was added thereto. After 30 minutes, these matrices were washed with 0.1M PBS and evaluated for the thrombus formation.

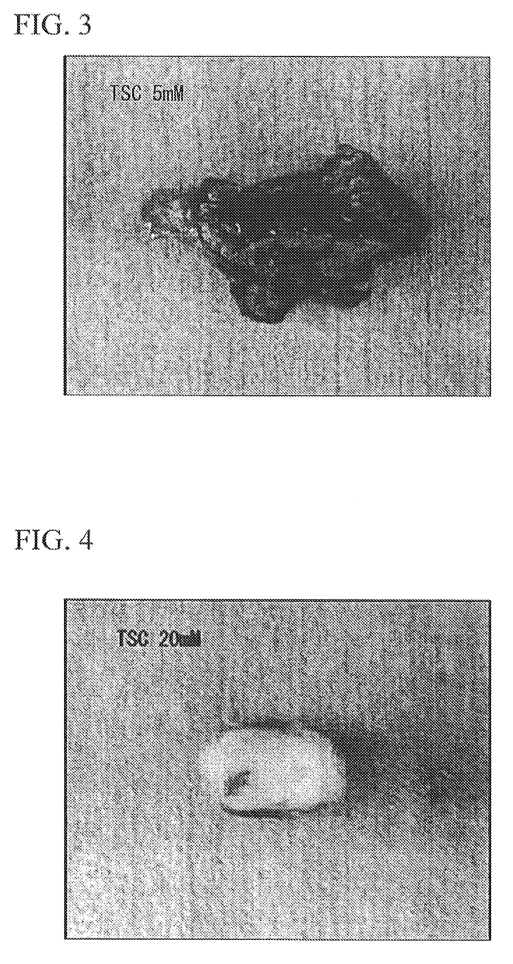

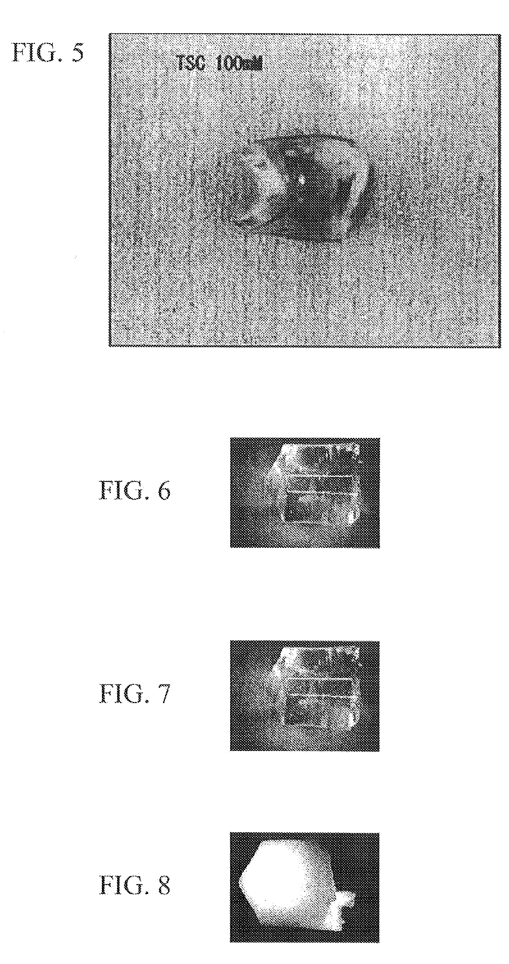

As shown in FIGs. 3 and 5, with the composite materials each having 5 mM and 100 mM organic acid derivative, thrombus formation was found on the surfaces of the high-molecular-weight matrices. On the other hand, as shown in FIG. 4, with the composite material having 20 mM organic acid derivative, almost no thrombus formation was found. It was suggested that this antithrombogenic activity might be caused by inhibiting the adsorption of platelet in blood, because active ester-derived carboxyl groups in the organic acid derivative TSC were more abundant than amino groups in gelatin and thus the high-molecular-weight matrix was negatively charged.

From the above-mentioned results, it was demonstrated to be possible, by controlling the concentration of the organic acid derivative, to form a high-molecular-weight matrix containing a low-molecular-weight organic compound and having an antithrombogenic activity on the surface, and to produce a composite material from which the amount of release of the low-molecular-weight organic compound is controllable.

### Example 7

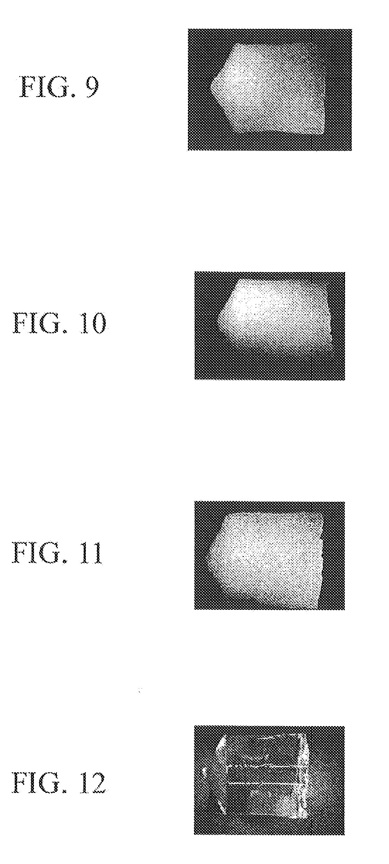

High-molecular-weight matrices formed from alkali-treated gelatin (AlGltn; derived from pig bone) and TSC (citric acid derivative), in which an amino acid derivative was contained, were prepared. By immersing in water, DMSO was replaced by water and the amino acid was precipitated. The behavior of the high-molecular-weight matrices after the precipitation was observed for each type and concentration of the amino acid.
Alkali-treated gelatin (AlGltn) derived from pig bone: Nitta gelatin Inc.
Citric acid derivative (TSC): Synthetic product
Amino acid derivative used: AcTrpNH₂, AcTyrNH₂, FmocIleOH, FmocPheOH, FmocTrpOH, and FmocSer(tBu)OH of Watanabe Chemical Industries, Ltd.
DMSO: SIGMA-ALDRICH, Inc.
L-lactic acid (LA): Wako Pure Chemical Industries, Ltd.
400 µl of a DMSO solution containing 15 w/v% AlGltn and 10% lactic acid was added with an amino acid derivative to give 100 mM in a 2 ml tube. The mixture was agitated and degassed. 100 µl of a 10% lactic acid-containing DMSO was added with TSC at 20 mM (4.84 mg) and agitated. The solution made by addition of the amino acid derivative to the AlGltn/10% lactic acid-containing DMSO solution, agitation, and degasification, was added with the TSC/10% lactic acid-containing DMSO solution. The mixture was agitated for 30 seconds and then degassed for 30 seconds. After leaving still for one day, the resulting high-molecular-weight matrix was taken out and immersed in water at 4°C for one day. The gel was taken out and its behavior was observed. Table 9 and FIGs. 6 to 12 show the presence or absence of the composite formation.

**[Table 9]**

| | 100 mM (dose of amino acid derivative) | Figure | Composite formation |
|---|---|---|---|
| AcTrpNH₂ | 12.26 mg | FIG. 6 | Absent |
| AcTyrNH₂ | 11.11 mg | FIG. 7 | Absent |
| FmocIleOH | 20.27 mg | FIG. 8 | Present |
| FmocPheOH | 19.37 mg | FIG. 9 | Present |
| FmocTrpOH | 21.32 mg | FIG. 10 | Present |
| FmocSer(tBu)OH | 19.17 mg | FIG. 11 | Present |
| Not contained | N/A | FIG. 12 | Absent |

### INDUSTRIAL APPLICABILITY

The present invention makes it possible, by using a biocompatible polymer and an organic acid derivative, to prepare a composite material of a high-molecular-weight matrix containing a low-molecular-weight organic compound. The rate of release of the low-molecular-weight organic compound from the composite material can be readily controlled by adjusting the crosslink density. Accordingly, a material whose drug releasing property is controllable and which is highly safe *in vivo* can be provided. This is extremely useful for the industry.

## Claims

1. A composite material comprising a high-molecular-weight matrix and a low-molecular-weight organic compound, wherein the composite material has the low-molecular-weight organic compound dispersed in the high-molecular-weight matrix, and the high-molecular-weight matrix is a polymer crosslinked with an organic acid derivative.

2. A composite material comprising a high-molecular-weight matrix and a low-molecular-weight organic compound according to claim 1, wherein the organic acid derivative has two or three carboxyl groups of a dicarboxylic acid or a tricarboxylic acid modified with electron attracting groups.

3. A composite material comprising a high-molecular-weight matrix and a low-molecular-weight organic compound according to claim 1 or claim 2, wherein the organic acid derivative is a single type of, or a combination of a plurality of types of, modified products in which at least two carboxyl groups of any one or more items selected from the group consisting of citric acid, malic acid, oxaloacetic acid, tartaric acid, cis-aconitic acid, 2-ketoglutaric acid, and derivatives thereof, are modified with N-hydroxysuccinimide, N-hydroxysulfosuccinimide, or a derivative thereof.

4. A composite material comprising a high-molecular-weight matrix and a low-molecular-weight organic compound according to any one of claim 1 through claim 3, wherein the polymer comprises a single item, or a combination of a plurality of items, selected from a protein, a glycosaminoglycan, a chitosan, a polyamino acid, and a polyalcohol.

5. A composite material comprising a high-molecular-weight matrix and a low-molecular-weight organic compound according to claim 4, wherein the glycosaminoglycan comprises a single item, or a plurality of items, selected from the group consisting of chondroitin sulfate, dermatan sulfate, hyaluronic acid, heparan sulfate, heparin, keratan sulfate, and derivatives thereof.

6. A composite material comprising a high-molecular-weight matrix and a low-molecular-weight organic compound according to claim 4, wherein the protein comprises a single item, or a combination of a plurality of items, selected from the group consisting of collagen, atelocollagen, alkali-treated collagen, gelatin, acid-treated gelatin, alkali-treated gelatin, keratin, serum albumin, egg albumin, recombinant albumin, hemoglobin, casein, globulin, fibrinogen, and derivatives thereof.

7. A composite material comprising a high-molecular-weight matrix and a low-molecular-weight organic compound according to any one of claim 1 through claim 6, wherein the low-molecular-weight organic compound comprises a single item, or a combination of a plurality of items, selected from: a drug which is soluble in an aprotic polar organic solvent, an amino acid, and a derivative thereof.

8. A composite material comprising a high-molecular-weight matrix and a low-molecular-weight organic compound according to any one of claim 1 through claim 7, wherein the composite material has the property of gradually releasing the low-molecular-weight compound.

9. A composite material comprising a high-molecular-weight matrix and a low-molecular-weight organic compound according to any one of claim 1 through claim 8, wherein the composite material has an antithrombogenic activity.

10. A process for producing a composite material comprising a high-molecular-weight matrix and a low-molecular-weight organic compound according to any one of claim 1 through claim 9, wherein the process comprises the steps of: crosslinking a polymer with an organic acid derivative in an organic solvent while, at the same time, embedding the low-molecular-weight organic compound therein; and substituting the organic solvent with water after the production of the high-molecular-weight matrix.
